(19) Europäisches Patentamt
European Patent Office
Office européen des brevets

(11) Numéro de publication: 0 097 551
B1

(12) # FASCICULE DE BREVET EUROPÉEN

(45) Date de publication du fascicule du brevet: **15.10.86**

(21) Numéro de dépôt: **83401091.0**

(22) Date de dépôt: **31.05.83**

(51) Int. Cl.[4]: **C 07 F 9/00**, C 07 F 9/22, C 07 F 9/54, B 01 J 31/36, C 07 C 27/16, C 07 C 37/60, C 07 D 301/03

(54) **Utilisation de complexes péroxydiques du vanadium, du niobium et du tantale comme réactifs et comme catalyseurs d'hydroxylation des hydrocarbures aromatiques.**

(30) Priorité: **21.06.82 FR 8210938**

(43) Date de publication de la demande: **04.01.84 Bulletin 84/1**

(45) Mention de la délivrance du brevet: **15.10.86 Bulletin 86/42**

(84) Etats contractants désignés: **BE DE GB NL**

(56) Documents cité:
**FR-A-2 489 710**

**JOURNAL OF MOLECULAR CATALYSIS, vol. 7, no. 1, 1980, pages 59-74, Elsevier Sequoia S.A., NL, O. BORTOLINI et al.: "Metal catalysis in oxidation by peroxides part 8. (1) Further insight on the mechanism of vanadium (V) catalyzed oxidation of sulphides and alkenes by hydrogen peroxide" TETRAHEDRON, vol. 26, no. 1, janvier 1980, pages 37-50, Pergamon Press, GB, H. MIMOUN et al.: "Epoxydation des olefines par les complexes peroxydiques covalents du molybdene-VI" INORGANIC CHEMISTRY, vol. 16, no. 6, juin 1977, pages 1349-1353, S. FUNAHASHI et al.: "Reactions of hydrogen peroxide with metal complexes. 2. Kinetic studies on the peroxo complex formation of nitrilotriacetatodioxovanadate (V) and dixo(2,6-pyridinedicarboxylato)vanadate(V)" INORGANIC CHEMISTRY, vol. 17, no. 1, janvier 1978, pages 57-64, American Chemical Society, USA, K. WIEGHARDT: "Preparation and**

(73) Titulaire: **INSTITUT FRANCAIS DU PETROLE, 4, Avenue de Bois- Préau, F-92502 Rueil- Malmaison (FR)**

(72) Inventeur: **Mimoun, Hubert, 34, rue Hippolyte Bisson, F-92500 Rueil- Malmaison (FR)**
Inventeur: **Saussine, Lucien, 27- 29, Av. de Brimon, F-78400 Chatou (FR)**
Inventeur: **Daire, Erick, 17, rue Michelet, F-92500 Rueil- Malmaison (FR)**
Inventeur: **Robine, Alain, 2, rue des Accacias, F-92140 Clamart (FR)**
Inventeur: **Guibourd de Luzinais, Jacques, 10, rue Masséna, F-92500 Rueil- Malmaison (FR)**

(56) Documents cité: (suite)
**characterization of dipicolinatovanadium (V) complexes. Kinetics and mechanism of their reaction with hydrogen peroxide in acidic media" JOURNAL OF THE CHEMICAL SOCIETY-DALTON TRANSACTIONS, vol. 11, 1973, pages 1137-1141, N. VULETIC et al.: "Oxodiperoxovanadate(V) complexes with bidentate ligands" JOURNAL OF THE AMERICAN CHEMICAL SOCIETY, vol. 105, no. 10, 18 mai 1983, pages 3101-3110, American Chemical Society, USA, H. MIMOUN et al.: "Vanadium (V) peroxo complexes. New versatile biomimetic reagents for epoxidation of olefins and hydroxylation of alkanes and aromatic hydrocarbons"**

**Le dossier contient des informations techniques présentées postérieurement au dépôt de la demande et ne figurant pas dans le présent fascicule.**

**EP 0 097 551 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La prèsente invention concerne l'utilisation de complexes péroxydiques du vanadium, du niobium et du tantale comme catalyseurs d'hydroxylation des hydrocarbures aromatiques en phénols correspondants.

Par complexes péroxydiques, on désigne les composés métalliques dans lesquels le métal est lié à une molécule d'oxygène porteuse de deux charges négatives ($O_2 2-$), ces complexes pouvant être de type péroxo (forme A) ou hydropéroxo (forme B)

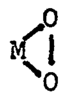

A

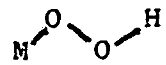

B

A B

Les complexes utilisables dans la présente invention ont l'une des formules suivantes I à III:

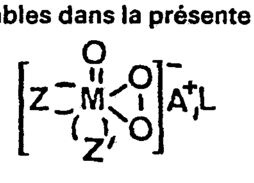

(I)

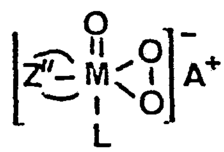

(II)

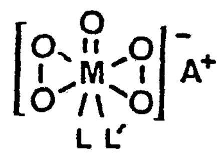

(III)

Dans les formules (I) à (III), M est le vanadium, le niobium ou le tantale, Z et Z', identiques ou différents, sont des composés mono ou polyaromatiques hétérocycliques azotés possédant un groupement carboxylique vicinal de l'azote, substitues ou non par au moins un groupement, Y choisi dans le groupe constitué par un groupement alkyle, aryle, alkoxy, aryloxy, nitro, halogène, carboxylique, ester, carboxyamide et hydroxyle, Z" est un ligand dianionique tridenté dérivé de l'acide pyridine 2,6 - dicarboxylique substitué par un ou plusieurs groupements Y, L et L' identiques ou différents, représentant chacun une molécule d'eau ou d'alcool ou un ligand mono ou bidendate à caractère éléctrodonneur, (ou représentant ensemble un ligand bidendate) ledit ligand étant choisi dans le groupe constitué par

a) une amine aromatique
b) un amide tertiaire linéaire ou cyclique
c) un phosphoramide
d) un oxyde d'amine aliphatique ou aromatique
e) un oxyde de phosphine, d'arsine ou de stibine,

A+ représentant un proton ou un cation ou un groupement cationique choisi dans le groupe constitué par

a) un métal alcalin
b) un groupe ammonium quaternaire
c) un groupe phosphonium ou arsonium quaternaire.

On trouve dans l'art antérieur des complexes de structures voisines décrits notamment dans le brevet fran*cais N° 2,489,710 (qui décrit divers composés du vanadium), dans la revue Inorganic Chemistry, volume 17, n° 1, pages 58 et 59 (où est étudié le comportement cinétique de complexes du vanadium analogues à certains de ceux que l'on décrit dans la présente demande de brevet), dans la revue Inorganic Chemistry, volume 16, n° 6, page 1351 (où sont également étudiés les comportements cinétiques de complexes du vanadium), dans Journal of Molecular Catalysis, 7, 1980, page 60, (voir formule 4) où l'effet du vanadium est étudié dans les oxydations catalytiques des sulfures et des alkynes, dans J. Chemical Soc. Dalton Trans., vol. 11, 1973, page 1137 où sont décrits des complexes oxodiperoxydiques du vanadium avec des ligands bidentés.

**I - DEFINITION PREFEREE DE Z, Z', Z", L, L' et A+**

1/ Dans les complexes précités (I), Z et Z' sont de préference des composés mono ou polyaromatiques hétérocycliques azotés ayant un groupement carboxylique vicinal de l'azote, substitués ou non par un ou plusieurs groupements Y, où Y représente un radical alkyle, aryle, alkyloxy, aryloxy, ou représente un groupe $NO_2$ ou un halogène, un groupe carboxylique, un ester, un carboxyamide, un hydroxyle, ces groupements Y pouvant occuper une ou plusieurs des positions quelconques du ou des noyaux aromatiques distincts ou accolés selon par exemple la formule représentative suivante:

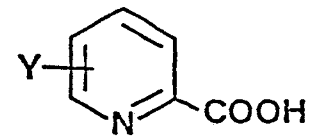

On peut citer à titre d'exemples non limitatifs de tels composés mono ou polyaromatiques, l'acide pyridine-2-carboxylique (ou picolinique), l'acide pyridine 2,3 -; 2,4 -; 2,5 - dicarboxylique, l'acide quinoléins 2 et 8 - carboxylique, l'acide isoquinoléine 1 - ou 3 - carboxylique, les acides 3, 4, 5 et 6 - chloro, bromo, nitro, hydroxy, méthyl, éthyl, propyl, butyl, phényl picolinique, les acides pyridine 2,3 -; 2,4 -; 2,5 - ou 2,6 - dicarboxylique mono alkyl ou aryl ester, les acides 1,2 -, 1,3 - ou 1,4 - pyrazine 6 -dicarboxylique.

On peut également utiliser, comme ligands anioniques bidentés Z et Z', les composés mono ou polyaromatiques hétérocycliques azotés N-oxyde ayant un groupement carboxylique vicinal de l'azote, substitués ou non par un ou plusieurs groupes Y choisis dans le groupe constitué par un groupement alkyle, aryle, alkyloxy, aryloxy, nitro, halogène, carboxylique, carboxyamide, hydroxyle, ces groupements Y pouvant occuper une ou plusieurs des positions quelconques du ou des noyaux aromatiques choisis, selon la formule

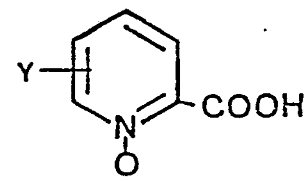

On peut citer, à titre d'exemples non limitatifs, l'acide picolinique N - oxyde, l'acide quinoléine 2 - ou 8 - carboxylique N - oxyde, les acides pyridine 2,3 -; 2,4 -ou 2,5-dicarboxylique N-oxyde.

2/ Dans la formule II, Z" est de préférence un ligand dianionique tridenté dérivé de l'acide pyridine 2,6-dicarboxylique (ou dipicolinique), mono, di ou trisubstitué par un ou plusieurs groupements Y, selon la formule

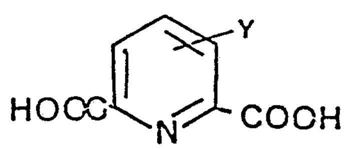

dans laquelle Y est un groupement alkyle aryle, alkyloxy, aryloxy, halogène, nitro, carboxylique, carboxyamide, hydroxyle.

3/ Dans les complexes (I) à (III), L et L' identiques ou différents représentent une molécule d'eau, un alcool tel que le méthanol ou un ligand mono ou bidendate (ou encore représentant ensemble un ligand bidendate) à caractère électrodonneur, ledit ligand étant choisi dans le groupe constitué par:

a) une amine aromatique comportant un ou plusieurs atomes d'azote par noyau. On peut citer, à titre d'exemples non limitatifs, la pyridine, la quinoléine, l'acridine, les 2 -, 3 - et 4 - picolines, la collidine, la 4 - dimethylaminopyridine, l'acide picolinique, le picolinate de méthyle, l'acide nicotinique, l'acide isonicotinique, le N - méthyl imidazole, la 2,2' - bipyridine, l'orthophénantroline et la pyridine-4-carboxylate de méthyle,

b) un amide tertiaire linéaire ou cyclique de formule $R''_1$ CO N $R''_2$ $R''_3$ dans laquelle $R''_1$, $R''_2$ et $R''_3$ identiques ou différents représentent chacun un radical hydrocarboné comportant de 1 à 20 atomes de carbone. On peut citer à titre d'exemples non limitatifs le diméthyl formamide, le diméthylacétamide, la N,N-diméthyl benzamide, la N,N - diéthylnicotinamide, la bis N,N - diéthylphtalimide, la N - acétyl morpholine, la N - benzoylpiperidine, la N - formyl piperidine, la N - acétyl piperidine, la N - méthylpyrrolidone, la N - éthylpyrrolidone, la N - phényl pyrrolidone, le N - méthyl valerolactame, le N - méthyl caprolactame, le N,N-diethyl picolinamide.

c) un phosphoramide de formule $(R'''_1\ R'''_2\ N)_3$ PO dans laquelle $R'''_1$, $R'''_2$, identiques ou différents représentent chacun un radical hydrocarboné comportant de 1 a 20 atomes de carbone par molécule. On peut citer, à titre non limitatif, l'hexaméthyl phosphorotriamide, l'hexaéthyl phosphorotriamide et l'octaméthylpyrophosphoramide.

d) un oxyde d'amine aliphatique ou aromatique tel que l'oxyde de triméthylamine, l'oxyde de N - méthylmorpholine, l'oxyde de pyridine, l'oxyde des 2 -, 3 - et 4 - picolines, l'oxyde de quinoléine, la 2,2' - bipyridine - N oxyde.

e) un oxyde de phosphine, d'arsine ou de stibine tel que la triphénylphosphine oxyde, la triphényl arsine oxyde, la triphénylstibine oxyde, la triméthyl phosphine oxyde, la méthyl diphényl phosphine oxyde, la diéthylphényl phosphine oxyde et la trimorpholinophosphine oxyde.

4/ Dans les complexes I à III, $A^+$ représente un proton ou un cation ou groupement cationique choisi dans le

groupe constitué, par exemple, par:

a) un métal alcalin tel que le lithium, le sodium, le potassium, le rubidium ou le césium,

b) un groupe ammonium quaternaire de forme générale N+ R R' R'' R''' dans laquelle R, R', R'' et R''' identiques ou différents sont chacun un atome d'hydrogène ou un radical hydrocarboné alkyle, aryle, aralkyle ou alkylaryle comprenant de 1 à 20 atomes de carbone. On peut citer à titre d'exemples non limitatifs le cation ammonium, les cations tétraméthyl, tétraéthyl, tétrapropyl, tétrabutyl, tétraphényl ammonium, les cations méthyltriphényl, benzyltriméthyl, benzyl tributyl, benzyltriphényl, cétyltriméthyl, cétyltriphényl ammonium,

c) un groupe phosphonium quaternaire de formule générale P R R' R'' R''' dans laquelle R, R', R'', R''', identiques ou différents, sont chacun un atome d'hydrogène ou un radical hydrocarboné alkyle, aryle, aralkyle ou alkylaryle comprenant de 1 à 20 atomes de carbone. On peut citer a titre d'exemples non limitatifs les cations tétraméthyl, tétraethyl, tétrapropyl, tétrabutyl, tétraphényl phosphonium, les cations méthyltributyl, méthyltriéthyl, méthyltriphényl, butyltriphényl, éthyltriphénylphosphonium,

d) un groupe arsonium quaternaire de formule générale As+ R R'R''R''' dans laquelle R, R', R'', R''' identiques ou différents sont chacun un radical hydrocarboné alkyle, aryle, aralkyle ou alkylaryle comprenant de 1 à 20 atomes de carbone. On peut citer à titre d'exemples non limitatifs les cations tétraméthyl, tétraéthyl, tétrapropyl, tétrabutyl et tétraphénylarsonium.

D'une fa*con générale, on résumera comme suit les radicaux ou groupements préférés des formules générales (I) à (III):

Z et Z' identiques ou différents sont choisis dans le groupe constitué par l'acide picolinique, l'acide pyrazine-2-carboxylique, l'acide quinoléine-2- carboxylique, l'acide pyridine 2,4-dicarboxylique et l'acide pyridine 2,5-dicarboxylique;

Z'' est l'acide pyridine 2,6-dicarboxylique;

L et L', identiques ou différents, sont chacun une molécule d'eau ou d'alcool, ou un ligand à caractère électrodonneur mono ou bidendate choisis dans le groupe constitué par la pyridine, la quinoléine, l'acridine, les 2-, 3- et 4- picolines, le picolinate de méthyle, la 2,2' bipyridine, l'orthophénantroline, le diméthylformamide, le diméthylacétamide, le diethylformamide, la N,N-diethylpicolinamide, la N,N - diethylpicolinamide, la N-méthyl pyrolidone, l'hexaméthylphosphotriamide, l'oxyde de N-méthylmorpholine, l'oxyde de pyridine, l'oxyde de 2 -, 3 -, et 4 - picolines, l'oxyde de quinoléine, la triphénylphosphine oxyde, la triphénylarsine oxyde et la pyridine-4-carboxylate de méthyle.

## II - PREPARATION DES COMPLEXES

Les préparations préférées des complexes, qui seront décrites de fa*con plus détaillée dans les exemples, peuvent s'effectuer de différentes fa*cons. Elle consistent généralement à faire réagir un sel ou complexe de vanadium niobium ou tantale avec l'eau oxygénée en présence des ligands Z H, Z'H et $Z''H_2$ précurseurs des anions Z, Z' et Z'' précités, en présence ou non des ligands électrodonneurs L et L' et en présence ou non des sels de formule $A^+ X^-$ (précurseurs des cations ou groupes cationiques A+) dans laquelle $X^-$ est un anion tel que par exemple le chlore, le brome, l'iode, un carboxylate, un nitrate, un perchlorate, un cyanure, etc...

Comme précurseur du vanadium, on peut choisir, par exemple, l'anhydride vanadique $V_2\ O_5$, l'acétylacétonate de vanadium V (acétyl-acétonate)$_3$ ou VO (acétylacétonate)$_2$, un alcoolate de vanadyle VO (OR)$_3$, le sulfate de vanadyle $VOSO_4$, le nitrate de vanadyle VO $(NO_3)_3$, le chlorure de vanadyle VO $Cl_3$ ou un alkyldithiocarbamate de vanadyle VO $(R_2NCS_2)_3$.

Comme précurseur du niobium et tantale, on peut choisir, par exemple, les oxydes $Nb_2\ O_5$ et $Ta_2\ O_5$, les chlorures Nb $Cl_5$ et Ta $Cl_5$, les oxychlorures $NbOCl_3$ et $TaOCl_3$, les niobates M'Nb $O_3$ et $M'TaO_3$ dans lesquels M' représente le lithium, le sodium, le potassium, le césium ou un cation ammonium.

Les différents constituants (précurseur métallique, ligands anioniqucs Z, Z', Z'' ligands neutres L et L', sels $A^+ X^-$, eau oxygénée) peuvent être introduits dans un ordre quelconque, selon des quantités égales ou différentes des rapports stoéchiométriques indiqués par les formules [I] à [III], en solution aqueuse, aquo-organique ou organique.

## III - REACTIVITE DES COMPLEXES (I) à (III).

Une des propriétés les plus caractéristiques des complexes est qu'ils peuvent céder stoéchiométriquement de l'oxygène à un substrat hydrocarboné pouvant être un hydrocarbure aromatique.

Dans le cas où le substrat est un hydrocarbure aromatique, le produit principal obtenu est le phénol correspondant selon l'équation

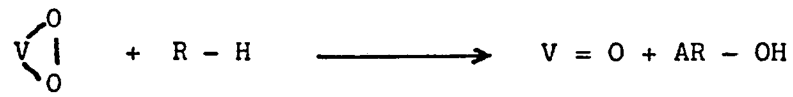 (1)

dans laquelle R représente un radical aromatique.

Une autre propriété caractéristique de ces complexes est qu'ils peuvent être régénérés à partir de leurs formes réduites correspondantes par addition d'un composé à caractère peroxydique tel que l'eau oxygénée ou un hydroperoxyde organique selon les équations (2) et (3):

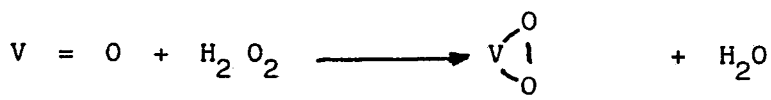 (2)

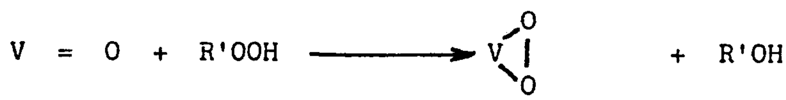 (3)

R' = radical organique.

Les complexes péroxydiques pourront donc être utilisés soit comme réactifs d'hydroxylation des hydrocarbures selon l'équation (1), soit comme catalyseurs d'oxydation, l'agent oxydant ultime étant l'eau oxygénée ou les hydroperoxydes organiques.

Une des propriétés remarquables des complexes (I) à (III) selon l'invention est de pouvoir céder de l'oxygène à des hydrocarbures aromatiques pour donner les phénols correspondants.

Les hydrocarbures aromatiques utilisables contiennent au moins un cycle benzénique à 6 atomes de carbone par molécule. Ces hydrocarbures aromatiques peuvent avoir de 1 à 6 cycles benzénique condensés, non condensés ou liés entre eux par des chaînes hydrocarbonées saturées renfermant par exemple 1 à 10 atomes de carbone. Les cycles aromatiques peuvent être non substitués, ou substitués par des groupements hydrocarbonés ou par un groupement choisi dans le groupe constitué par les groupements méthoxy, carboxy, hydroxy, amino, chloro, bromo, nitro, cyano etc... Parmi les hydrocarbures aromatiques, on peut citer, par exemple, le benzène, le toluène, les ortho, méta et paraxylènes, le mésitylène, le durène, l'éthylbenzène, le cumène, le tertiobutylbenzène, les diisopropylbenzènes, le cymène, le naphtalène, l'anthracène, le phenantrène, l'anisole, l'acide benzoïque, le phénol, l'acétanilide, le chlorobenzène, le bromobenzène, le nitrobenzène, etc...

L'hydroxylation des hydrocarbures aromatiques par les complexes précités conduit aux phénols comme produits principaux. Cette hydroxylation s'effectue de préférence sur les positions les plus riches en électrons. Par exemple, le toluène est hydroxylé préférentiellement en ortho et paracrésol. Le benzène est hydroxylé en phénol.

Les complexes peuvent se comporter à la fois comme réactifs stoéchiométriques et comme catalyseurs d'oxydation en présence d'un donneur d'oxygène tel que l'eau oxygénée ou un hydroperoxyde organique.

Ils peuvent être mis en oeuvre directement dans le substrat hydrocarboné à oxyder ou dans un solvant organique quelconque dans lesquels ils sont solubles. A titre d'exemples de solvant, on citera, par exemple

- les solvants chlorés tels que le chlorure de méthyléne, le dichloroéthane, le chloroforme, le chlorobenzène, etc...
- les solvants nitrés tels que le nitrométhane, le nitrobenzène etc...
- les nitriles tels que l'acétonitrile, le propionitrile, le benzonitrile, etc...
- les cétones telles que l'acétone ou la méthyl-éthylcétone
- les solvants basiques pouvant être à base des groupements L et/ou L' définis plus haut, par exemple le diméthylformamide, le diméthylacétamide, la N-méthylpyrrolidone, l'hexaméthyl phosphorotriamide, la pyridine, la quinoléine, la pyridine N-oxyde.

Les complexes peuvent être utilisés tels quels en phase homogène. Ils peuvent être également déposés sur un support tel que la silice, l'alumine, les alumino silicates, les zéolithes, les charbons l'oxyde de titane le quartz, etc...

La réaction est effectuée à une température comprise generalement et par exemple entre - 50 et + 150° C, mais de préférence à une température comprise entre 0 et + 100° C.

La présence d'eau dans le milieu, bien que tolérable à concentration peu élevée, peut cependant être

genante et peut necessiter d'être enlevée, au moins en grande partie, par exemple par distillation azéotropique en présence d'un solvant tel par exemple que le benzène, le toluène ou le xylène, ou grâce à un agent desséchant ou tout autre moyen équivalent.

Le milieu réactionnel peut être neutre, acide ou basique. Il peut être avantageux dans certains cas d'opérer en présence d'un acide de Brönstedt dans le milieu, afin d'augmenter l'activité des complexes vis-à-vis des substrats hydrocarbonés.

Les acides utilisés peuvent être des acides carboxyliques, par exemple, l'acide acétique, l'acide propionique, l'acide benzoïque, ou des acides sulfoniques, par exemple l'acide méthane sulfonique, l'acide fluorosulfonique, l'acide paratoluène sulfonique.

Le rapport acide de Brönstedt sur métal peut être compris par exemple entre 1 et 10, mais on choisira généralement un rapport voisin de 1: 1.

L'oxydation des substrats hydrocarbonés peut s'effectuer stoéchiométriquement selon l'équation (1).

Elle peut également s'effectuer catalytiquement, c'est-à-dire avec de faibles quantités de complexe par rapport au substrat insaturé, lorsque l'on opère en présence d'un agent oxydant tel que l'eau oxygénée ou un hydroperoxyde organique.

Le rapport molaire complexe/substrat est généralement compris entre $10^{-4}$ et 0,5, voire entre $10^{-3}$ et $10^{-1}$.

Le rapport peroxyde/substrat est généralement compris entre 0,1 et 10, plus particulièrement entre 0,1 et 2.

Lorsque l'eau oxygénée est l'agent oxydant, on peut opérer en milieu biphasique, ou en milieu monophasique grâce à un tiers solvant tel que, par exemple, un alcool (par exemple le tert-butanol), un nitrile (par exemple l'acétonitrile), une cétone (par exemple l'acétone) ou un amide (par exemple le diméthyl formamide)

Lorsque l'hydroperoxyde organique est l'agent oxydant, on opère, généralement en phase organique homogène, dans un des solvants définis plus haut.

Comme hydroperoxyde de formule générale ROOH, on choisira de préférence l'hydroperoxyde de tertiobutyle, l'hydroperoxyde d'éthylbenzène ou l'hydroperoxyde de cumène.

La présente invention est illustrée par les exemples suivants:

**Exemple 1**

Préparation des complexes ioniques de type [I] de formule $[MO(O_2)ZZ'L]^- A^+$

a) Préparation des complexes 1, 2 et 3 du vanadium, de formule $[(Pic)_2VO(O_2)]^- A^+$, L (Pic = picolinate)

complexe 1: $A^+ = H^+$, $L = H_2O$ >>> Complexe 2: $A^+ = H^+$, L = HMPT

complexe 3: $A^+ = P(C_6H_5)_4^+$, $L = H_2O$

**Préparation du complexe n° 1**

On laisse réagir 4,52 g d'anhydride vanadique $V_2O_5$ (50 mM) et 12,3 g d'acide picolinique (100 mM) dans 20 cc d'eau oxygénée à 30 % pendant 4 heures à 0°C, jusqu'à dissolution complète du $V_2O_5$, et précipitation d'un précipité rouge brique.

Ce précipité est filtré, lavé à l'éther et séché sur $P_2O_5$.

On recueille 17 g de complexe rouge brique 1 (rendement molaire = 93 %) dont les analyses élémentaires, infra-rouge, RMN du proton indiquent la formule

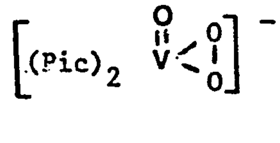 $H^+$, $H_2O$

Analyse élémentaire: calculée pour $VC_{12}H_{10}N_2O_8$

C = 39,7; H = 3,03; N = 7,73; Trouvée:

C = 40,5; H = 2,95; N = 7,85

Infrarouge = $\nu(V = 0) = 965\ cm^{-1}$; $\nu(O - 0) = 935\ cm^{-1}$

**Préparation du complexe n° 2**

On reprend la même synthèse que pour le complexe 1 (4,52 g de $V_2O_5$, 12,3 g d'acide picolinique et 20 cc $H_2O_2$ à 30%) mais en ajoutant en plus 13,5 g d'hexaméthyl phosphorotriamide (75 m moles).
Le complexe formé 2 est recueilli par extraction dans le chlorure de méthylène et précipitation à l'éther.
Analyse élémentaire: calculée pour $VC_{18}H_{26}N_5O_8P$
V = 9, 75; C = 41,3; H = 5,16; N = 13,38; Trouvée:
V = 9,7; C = 41,4; H = 5,18; N = 13,3
Infra-rouge = (V = 0) = 965 $cm^{-1}$; (0 - 0) = 940 $cm^{-1}$

**Préparation du complexe n° 3**

A une solution de $NH_4$ $VO_3$ (1,17 g = 10 mM), et d'acide picolinique (2,46 g = 20 mM) dans 10 cc d'$H_2O_2$ à 30 % on ajoute 4,2 g de bromure de tétraphénylphosphonium dissous dans le minimum de méthanol. Le complexe 3 précicipe, et il est recueilli par filtration, puis lavé à l'éther; rendement: 6,5 g (92 % molaire).
Analyse élémentaire: calculée pour $VC_{36}H_{30}N_2O_8P$
C = 61,7; H = 4,28; N = 4,0; P = 4,43
Trouvée: C = 61,3; H = 4,21; N = 4,01; P = 4,39
Infra-rouge: (V = 0) = 960 $cm^{-1}$, (0 -0) = 935 $cm^{-1}$
Le même complexe a été obtenu avec de bons rendements molaires (> 80%) par addition d'une quantité stoechiométrique de bromure de tétraphénylphosphonium à une solution du complexe 1 ou du complexe 2 dans l'eau.
b) Préparation des complexes 4, 5 et 6 du niobium de formule $[(Pic)_2 NbO (O_2)]^- A^+$, L
L = $H_2O$
complexe 4: A+ = $P (C_6 H_5)_4$
complexe 5: A+ = $P (C_4 H_9)_4$
complexe 6: A+ = $P (C_4 H_9) (C_6H_5)_3$

**Préparation du complexe n° 4**

5,04 g de niobate de potassium $KNbO_3$ (20 mM) et 4,92 g d'acide picolinique (40 mM) sont dissous dans 30 cc d'eau oxygénée à 30 %. L'addition de 8,4 g de bromure de tétraphénylphosphonium (20 mM), dissous dans le minimum de méthanol, provoque la précipitation du complexe jaune pâle 4. Rendement après lavage et séchage: 14 g (94 % molaire). Analyse élémentaire = calculée pour Nb $O_7C_{36}H_{28}P$ $N_2$
C = 59,6. H = 3,86; N = 3,86; O = 15,46
Trouvée: C = 58,5; H = 3,85; N = 3,84: O = 15.56
Infra-rouge: ν(Nb = 0) = 850 $cm^{-1}$ ν(0 - 0) = 875 cm $^{-1}$

**Préparation du complexe n° 5**

Ce complexe a été préparé de la même fa•con que le complexe 4, mais en utilisant le bromure de tétrabutylphosphonium.
Le complexe 5 est extrait de la solution au chlorure de méthylène puis purifié.
rendement molaire = 90 %.
Infra-rouge ν(Nb = 0) = 850 $cm^{-1}$ ν(0 = 0) = 875 $cm^{-1}$

**Préparation du complexe n° 6**

Même préparation que le complexe 4, mais en utilisant le bromure de butyl triphényl phosphonium. Le complexe 6 formé précipite; rendement molaire: 85 %.
Analyse élémentaire: calculée pour Nb $O_7C_{34}H_{32}P$ $N_2$
C = 57,9: H = 4,54; N = 3,97; Trouvée:
C = 57,2; H = 4,54; N = 3,92
c) Préparation des complexes 7 et 8 du tantale de formule $[(Pic)_2 Ta O (O_2)]^- A^+$, L
complexe 7: A+ = $P (C_6 H_5)_4$, L = $H_2O$
complexe 8: A+ = $P (CH_3) (C_6 H_5)_3$, L = $H_2O$

**Préparation du complexe n° 7**

720 mg de chlorure de tantale Ta $Cl_5$ (4 mM) sont dissous dans le minimum de méthanol. On ajoute ensuite 1 g (8 m M) d'acide picolinique solide, puis 10 cc d'eau oxygénée. On laisse agiter jusqu'à obtention d'une solution homogène. Puis on ajoute 1,68 g (4 mM) de bromure de tétraphénylphosphonium dissous dans l'eau. Le complexe 7, jaune pâle, précipite. Rendement après filtration, lavage à l'éther, et séchage sur $P_2O_5$: 80 % molaire.
Analyse élémentaire: calculée pour Ta $O_8$ $C_{36}$ $H_{30}$P $N_2$
C = 52,04; H = 3,61; N = 3,37; Trouvée:
C = 51,7; H = 3,6; N = 3,38
Infrarouge ν(Ta = 0) = 855 $cm^{-1}$ ν(0 - 0) = 835 $cm^{-1}$

**Préparation du complexe n° 8**

même préparation que pour le complexe 7 mais en utilisant le bromure de méthyltriphénylphosphonium. Rendement molaire: 80 %.
Infrarouge ν(Ta = 0)= 855 $cm^1$ ν(0 - 0) = 835 $cm^{-1}$

**Exemple 2**

Préparation des complexes ioniques de type II de formule [Z'' MO ($O_2$)]$^-$ $A^+$,
complexe 9: Z'' = pyridine - 2,6-dicarboxylate; M = vanadium
L = $H_2O$; A = P ($C_6$ $H_5$)$_4$
complexe 10: Z'' = pyridine - 2,6 dicarboxylate; M = niobium
L = $H_2O$; A = P ($C_6$ $H_5$)$_4$

**Préparation du complexe 9**

1,82 g d'anhydride vanadique (20 m M) et 3,4 g (20 mM) d'acide pyridine 2,6 dicarboxylique sont dissous à 70° C dans 10 cc d'eau. A la solution jaune pâle limpide refroidie à 0° C, on ajoute 2 cc d'$H_2O_2$ à 30 % puis 8,4 g (20 mM) de bromure de tetraphénylphosphonium. Le complexe 9 précipite; il est filtré, lavé à l'éther et séché; rendement: 90% molaire.
Infrarouge ν(V = 0) = 960 $cm^{-1}$ ν(0 - 0) = 935 $cm^{-1}$

**Préparation du complexe 10**

2,52 g de niobate de potassium (10 m M) et 1,7 g d'acide dipicolinique (10 mM) et 1,7 g d'acide dipicolinique (10 mM) sont dissous dans 20 cc d'eau oxygénée à 30 %. A la solution jaune limpide obtenue, on ajoute 4,2 g de bromure de tetraphénylphosphonium (10 mN). Le complexe 10 qui précipite est recueilli par filtration; rendement: 85 % molaire.
Infrarouge ν(Nb = 0) = 850 $cm^{-1}$, ν(0 - 0) = 875 $cm^{-1}$

**Exemples 3 à 5**

Hydroxylation stoechiométrique des hydrocarbures aromatiques.
Dans un réacteur en verre calorifugé, on introduit sous azote 0,04 0 mol.$1^{-1}$ d'un des complexes 1 ou 2, 2 mol.$1^{-1}$ de substrat hydrocarboné aromatique dans un des solvants figurant dans le tableau II.
La température est de 20° C. Au bout de 4 h, on analyse les produits formés par chromatographie en phase gazeuse que l'on identifie par couplage chromatographie en phase gazeuse - spectrométrie de masse.
Les résultats obtenus sont rassemblés dans le tableau II. Les rendements sont exprimés par rapport au vanadium.

**Tableau II**

Hydroxylatiòn des hydrocarbures aromatiques
Exemple N°:complexe Solvant Substrat Produit(s) Rdt molaire %
3 1 $CH_3CN$ Benzène Phénol (43)
4 2 " " " (30)
5 1 " Toluène o-cresol (8) p+m cresol(14)

**Exemple 6**

Hydroxylation catalytique du benzène en phénol par l'eau oxygénée

Dans un réacteur calorifugé et agité, muni d'un réfrigérant on introduit 100 ml de benzène, puis 0,1 m mole de catalyseur. Le réacteur est porté au reflux du benzène à 80°C. L'eau oxygénée à 70 % (10 ml) est alors introduite goutte à goutte de fa•con à maintenir une phase aqueuse très faible, compte tenu de l'élimination d'eau par distillation azéotropique.

La quantité de phénol produite est déterminée par chromatographie en phase gazeuse.
Catalyseur phénol
N° Complexe m mole Temps de réaction
9 0,5 0,5 h

**Exemples 7 à 9**

Hydroxylation catalytique du benzène en phénol par l'hydroperoxyde de tert-butyle

Dans un réacteur calorifugé maintenu à 60°C, on introduit 20 ml de benzène, 0,1 m mole de catalyseur et 20 m moles d'hydroperoxyde de tert-butyle.

La quantité de phénol produite est déterminée par chromatographie en phase gazeuse, et celle d'hydroperoxyde non consommé par iodométrie.
Exemples N° complexe Phénol durée
(m moles)
7 9 0,3 0,5 h
8 2 0,4 "
9 1 0,4 "

**Revendications**

1/ Procédé d'hydroxylation d'hydrocarbures aromatiques en présence d'un réactif ou d'un catalyseur à base d'au moins un complexe péroxydique du vanadium ou du niobium ou du tantale choisi dans les formules générales I à III.

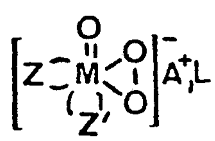
(I)

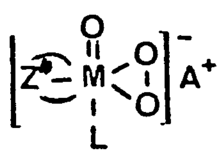
(II)

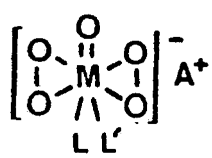
(III)

dans lesquelles M représente le vanadium, le niobium, ou le tantale, Z et Z' identiques ou différents sont des composés mono ou polyaromatiques hétérocycliques azotés possédant un groupement carboxylique vicinal de l'azote, substitués ou non par au moins un groupement Y choisi dans le groupe constitué par un groupement alkyle, aryle, alkyloxy, aryloxy, nitro, halogène, carboxylique, carboxyamide et hydroxyle, Z'' est un ligand dianionique tridenté dérivé de l'acide pyridine 2,6 - dicarboxylique substitué par un ou plusieurs groupements Y, L et L' identiques ou différents, représentant chacun une molécule d'eau ou d'alcool ou un ligand mono ou bidendate à caractère éléctrodonneur, (ou représentant ensemble un ligand bidendate) ledit ligand étant choisi dans le groupe constitué par
a) une amine aromatique
b) un amide tertiaire linéaire ou cyclique
c) un phosphoramide
d) un oxyde d'amine aliphatique ou aromatique

e) un oxyde de phosphine, d'arsine ou de stibine,

A+ représentant un proton ou un cation ou un groupement cationique choisi dans le groupe constitué par

a) un métal alcalin

b) un groupe ammonium quaternaire

c) un groupe phosphonium ou arsonium quaternaire.

2/ Procédé selon la revendication 1 dans lesquels Z et Z' identiques ou différents ont choisis dans le groupe constitué par l'acide picolinique, l'acide pyrazine-2-carboxylique, l'acide quinoléine-2-carboxylique, l'acide quinoléine-2- carboxylique, l'acide pyridine2,4-dicarboxylique et l'acide pyridine 2,5-dicarboxylique.

3/ Procédé selon la revendication 1 dans lesquels Z" est l'acide pyridine 2,6-dicarboxylique.

4/ Procédé selon la revendication 1 dans lesquels L et L' identiques ou différents sont chacun une molécule d'eau ou d'alcool, ou un ligand à caractère électrodonneur mono ou bidendate choisis dans le groupe constitué par la pyridine, la quinoléine, l'acridine, les 2-, 3- et 4- picolines, le picolinate de méthyle, la 2,2'-bipyridine, l'orthophénantroline, le diméthylformamide, le diméthylacétamide, le diéthylformamide, la N,N-diethylpicolinamide, la N,N-diethylpicolinamide, la N-méthyl pyrolidone, l'hexaméthylphosphotriamide, l'oxyde de N-méthylmorpholine, l'oxyde de pyridine, l'oxyde de 2-, 3-, et 4- picolines, l'oxyde de quinoléine, la triphénylphosphine oxyde, la triphénylarsine oxyde et la pyridine-4- carboxylate de méthyle.

5/ Procédé selon l'une des revendications 1 à 4, dans lequel on utilise au moins l'un des complexes I à III à titre de réactif dans une réaction d'hydroxylation d'hydrocarbures aromatiques en phénols.

6/ Procédé selon l'une des revendications 1 à 4 dans lequel on utilise au moins l'un des complexes I à III à titre de catalyseurs pour l'hydroxylation des hydrocarbures aromatiques par l'eau oxygénée ou les hydroperoxydes organiques.

7/ Procédé selon la revendication 6 dans lequel le rapport molaire complexe/substrat hydrocarboné est compris entre $10^{-4}$ et 0,5.

8/ Procédé selon l'une des revendications 1 à 7 appliqué à l'hydroxylation du benzène ou de toluène.

## Patentansprüche

1. Verfahren zur Hydroxylierung von aromatischen Kohlenwasserstoffen in Gegenwart eines Reagens oder eines Katalysators auf der Basis von zumindest einem Peroxo-Komplex von Vanadium oder Niob oder Tantal, ausgewählt aus den allgemeinen Formeln I bis III

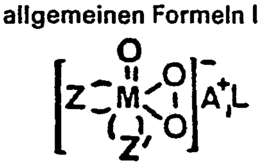

(I)

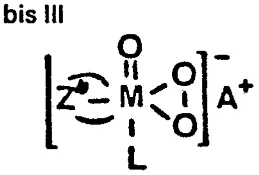

(II)

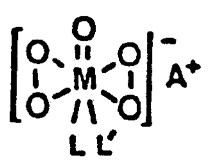

(III)

worin M Vanadium, Niob oder Tantal bedeutet Z und Z', gleich oder verschieden sein können, stickstoffhaltige heterozyklische mono- oder polyaromatische Verbindungen bedeuten, die eine zum Stickstoff vizinale Carboxylgruppe aufweisen und die gegebenenfalls durch zumindest eine Gruppe Y, die aus Alkyl-, Aryl-, Alkyloxy-, Aryloxy-, Nitro-, Halogen-, Carboxyl-, Ester-, Carboxamid- und Hydroxylgruppen ausgewählt sein kann, substituiert sein können, Z" einen dreizähnigen dianionischen Liganden bedeutet, der von Pyridin 2,6 - Dicarbonsäure abgeleitet ist, die durch eine oder mehrere Gruppen Y substituiert ist, L und L', die gleich oder verschieden sein können, jeweils ein Molekül Wasser oder Alkohol oder einen ein- oder zweizähnigen Liganden mit Elektronendonatorcharakter (oder zusammen einen zweizähnigen Liganden) bedeuten, wobei dieser Ligand aus der folgenden Gruppe ausgewählt ist

a) ein aromatisches Amin

b) ein tertiäres lineares oder zyklisches Amid

c) ein Phosphoramid

d) ein Oxid eines aliphatischen oder aromatischen Amins

e) ein Oxid von Phosphin, Arsin oder Stibin,

A+ ein Proton oder ein Kation oder eine kationische Gruppe bedeutet, die ausgewählt ist aus der Gruppe

a) ein Alkalimetall

b) eine quaternäre Ammoniumgruppe

c) eine quaternäre Phosphonium- oder Arsoniumgruppe.

2. Verfahren nach Anspruch 1, dadurch gekennzeichnet, daß Z und Z', die gleich oder verschieden sein können, aus der Gruppe Picolinsäure, Pyrazin-2-carbonsäure, Chinolin-2-carbonsäure, Pyridin-2,4-dicarbonsäure und Pyridin-2,5-dicarbonsäure gewählt sind.

3. Verfahren nach Anspruch 1, dadurch gekennzeichnet, daß Z" Pyridin-2,6-dicarbonsäure ist.

4. Verfahren nach Anspruch 1, dadurch gekennzeichnet, daß L und L', die gleich oder verschieden sein können, jeweils ein Molekül Wasser oder Alkohol sind oder ein ein- oder zweizähniger Ligand mit

Elektronendonatoreigenschaften, ausgewählt aus der Gruppe Pyridin, Chinolin, Acridin, 2-, 3- und 4-Picolin, Methylpicolinat, 2,2'-Bipyridin, Orthophenantrolin, Dimethylformamid, Dimethylacetamid, Diäthylformamid, N,N-Diäthylpicolinamid, N-Methylpyrrolidon, Hexamethylphosphotriamid, N-Methylmorpholinoxid, Pyridinoxid, 2-, 3- und 4-Picolinoxid, Chinolinoxid, Triphenylphosphinoxid, Triphenylarsinoxid und Pyridin-4-methylcarboxylat ist.

5. Verfahren nach einem der Ansprüche 1 bis 4, dadurch gekennzeichnet, daß man zumindest einen der Komplexe I bis III als Reagens für die Hydroxylierungsreaktion von aromatischen Kohlenwasserstoffen zu Phenolen verwendet.

6. Verfahren nach einem der Ansprüche 1 bis 4, dadurch gekennzeichnet, daß man zumindest einen der Komplexe I bis III als Katalysator für die Hydroxylierung von aromatischen Kohlenwasserstoffen mit Wasserstoffperoxid oder organischen Hydroperoxiden verwendet.

7. Verfahren nach Anspruch 6, dadurch gekennzeichnet, daß das molare Verhältnis Komplex/Kohlenwasserstoffsubstrat zwischen $10^{-4}$ und 0,5 liegt.

8. Verfahren nach einem der Ansprüche 1 bis 7, angewandt auf die Hydroxylierung von Benzol oder Toluol.

**Claims**

1.- Process for hydroxylation aromatic hydrocarbons in the presence of a reactant or of an oxidation catalyst containing vanadium or niobium or tantalum peroxidic complexes selected from the general formulas I to III

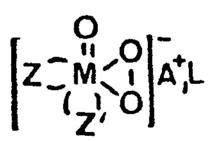 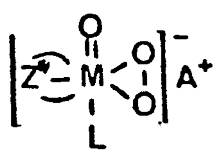 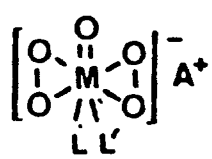

wherein M is vanadium, niobium or tantalum, Z and Z', identical or differens, are mono or polyaromatic nitrogenous heterocyclic compounds having a carboxylic group vicinal to the nitrogen atom, substituted or not by at least one Y group selected from the alkyl, aryl, alkyloxy, aryloxy, nitro, halogen, carboxylic ester, carboxyamide and hydroxyl groups, Z" is a dianionic tridentate ligand derived from 2,6-pyridine dicarboxylic acid substituted by one or more Y groups L and L' identical or different being each a molecule of water or alcohol or a mono or bidentate ligand of electrodonor character, (or forming together a bidentate ligand), said ligand being selected from the group constituted of

a) - an aromatic amine
b) - a linear or cyclic tertiary amide
c) - a phosphoramide
d) - an aliphatic or aromatic amine oxide
e) - a phosphine, arsine or stibine oxide,

A+ being a proton or a cation or a cationic group selected from:

a) - an alkali metal
b) - a quaternary ammonium group
c) - a phosphonium or arsonium quaternary group.

2.- The use according to claim 1, wherein Z and Z', identical or different, are selected from the group consisting of picolinic acid, 2-pyrazine carboxylic acid, 2-quinoline carboxylic acid, 2,4-pyridine dicarboxylic acid and 2,5-pyridine dicarboxylic acid.

3.- The use according to claim 1, wherein Z" is 2,6 pyridine dicarboxylic acid.

4.- The use according to claim 1, wherein L and L', identical or different, are each a molecule of water or alcohol, or a mono or bidendate ligand of electrodonor character selected from the group consisting of pyridine, quinoline, acridine, 2, 3 and 4-picolines, methyl picolinate, 2,2'-bipyridine, orthophenantroline, dimethylformamide, dimethylacetamide, diethylformamide, N,N-diethylpicolinamide, N-methylpyrrolidone, hexamethylphosphotriamide, N-methylmorpholine oxide, pyridine oxide, 2, 3- and 4-picoline oxides, quinoline oxide, triphenylphosphine oxide, triphenylarsine oxide and 4-pyridine methyl carboxylic ether.

5.- The use according to any of claims 1 to 4, of at least one complexe I to III, as reactants in a reaction of hydroxylating aromatic hydrocarbons to phenols.

6.- The use according to any of claims 1 to 4 of at least one of complexes I to III, as catalysts for hydroxylating aromatic hydrocarbons by hydrogen peroxide or organic hydroperoxides.

7.- A process according to any of claims 1 to 6, the molar ratio complex/hydrocarbon substrate being comprised between $10^{-4}$ and 0.5.

8.- The process according to any of claims 1 to 7, for hydroxylating benzene or toluene.